(19) Europäisches Patentamt
European Patent Office
Office européen des brevets

(11) EP 0 812 363 B1

(12) EUROPÄISCHE PATENTSCHRIFT

(45) Veröffentlichungstag und Bekanntmachung des Hinweises auf die Patenterteilung:
**24.01.2001 Patentblatt 2001/04**

(21) Anmeldenummer: **96904087.2**

(22) Anmeldetag: **01.03.1996**

(51) Int Cl.$^7$: **C12P 41/00**, C12P 13/00

(86) Internationale Anmeldenummer:
**PCT/EP96/00835**

(87) Internationale Veröffentlichungsnummer:
**WO 96/27022 (06.09.1996 Gazette 1996/40)**

# (54) VERFAHREN ZUR HERSTELLUNG VON OPTISCH AKTIVEN AMINEN

PROCESS FOR PREPARING OPTICALLY ACTIVE AMINES

PROCEDE DE PREPARATION D'AMINES OPTIQUEMENT ACTIVES

(84) Benannte Vertragsstaaten:
**AT BE CH DE DK ES FI FR GB IT LI NL**

(30) Priorität: **02.03.1995 DE 19507217**

(43) Veröffentlichungstag der Anmeldung:
**17.12.1997 Patentblatt 1997/51**

(73) Patentinhaber: **BAYER AG**
**51368 Leverkusen (DE)**

(72) Erfinder:
- **SMIDT, Hauke**
**NL-6701 Wageningen (NL)**
- **FISCHER, Andreas**
**D-79111 Freiburg (DE)**
- **FISCHER, Peter**
**D-70569 Stuttgart (DE)**
- **SCHMID, Rolf, D.**
**D-70191 Stuttgart (DE)**
- **STELZER, Uwe**
**D-51399 Burscheid (DE)**

(56) Entgegenhaltungen:
**EP-A- 0 399 589**

- **BIOORG. MED. CHEM., Bd. 2, Nr. 2, 1994, Seiten 429-432, XP002023593 J. OGAWA ET AL.: "Enzymatic Asymmetric Synthesis of Alfa-Methyl Arylalkylamines and Alfa-Methyl Arylalkylalcohols by Arylalkyl Acylamidases"**
- **J. ORG. CHEM., Bd. 44, Nr. 13, 1979, Seiten 2222-2225, XP002023594 D. ROSSI ET AL.: "Approach to the Use of Benzylpenicillinacylase for Configurational Correlations of Amino Compounds. 2. Hydrolysis of N-(p-Aminophenylacetyl) Derivatives of Some Chiral Primary Amines"**
- **PATENT ABSTRACTS OF JAPAN vol. 014, no. 306 (C-0735), 3.Juli 1990 & JP 02 104295 A (DAICEL CHEM IND LTD), 17.April 1990,**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein neues Verfahren zur Herstellung von (R)-Aminen durch enzymatische Hydrolyse von N-Acylamin-Racematen.

Es ist bereits bekannt geworden, optisch aktive N-p-Aminophenyl-acetylamino-Derivate durch enzymatische Spaltung der entsprechenden Racemate mit Benzylpenicillin-acylase als Biokatalysator herzustellen (vgl. J. Org. Chem. 44, 2222-2225 (1979). Ungünstig an diesem Verfahren ist aber, daß die optischen Ausbeuten zum Teil sehr niedrig sind. Außerdem wird bei dieser Methode bevorzugt das (S)-Enantiomere des Ausgangsracemates hydrolysiert. So entstehen zum Beispiel aus dem entsprechenden Racemat das (R)-Enantiomere von N-p-Amino-1-phenylacetyl-1-phenethylamin und das freie (S)-Amin. Ein weiterer Nachteil besteht darin, daß der Biokatalysator sehr substratspezifisch reagiert und nur solche Verbindungen einsetzbar sind, die eine Phenylacetyl-Gruppe enthalten.

Weiterhin ist bekannt, daß sich (S)-Amine und (R)-Enantiomere von N-acylierten 1-Methyl-1-phenyl-alkylaminen herstellen lassen, indem man Racemate von N-Acyl-1-methyl-1-phenyl-alkylaminen mit Hilfe bestimmter Biokatalysatoren umsetzt (vgl. EP-A 0 399 589). Nachteilig an diesem Verfahren ist, daß die korrespondierenden (R)-Amine nur erhältlich sind, wenn man die (R)-Enantiomeren der N-Acyl-1-methyl-1-phenyl-alkylamine in einem zusätzlichen Reaktionsschritt deacyliert.

Es wurde nun gefunden, daß man (R)-Amine der Formel

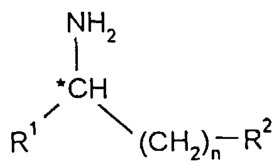 (I),

in welcher

$R^1$ für geradkettiges oder verzweigtes Alkyl mit 1 bis 8 Kohlenstoffatomen steht, wobei die Alkylreste einfach oder mehrfach, gleichartig oder verschieden substituiert sein können durch
Halogen, Cyano, Amino, Hydroxy, Formyl, Carboxy, Carbamoyl, jeweils geradkettiges oder verzweigtes Alkoxy oder Alkylthio mit jeweils 1 bis 6 Kohlenstoffatomen;
jeweils geradkettiges oder verzweigtes Halogenalkoxy oder Halogenalkylthio mit jeweils 1 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
oder durch zweifach verknüpftes Alkylen mit 1 bis 6 Kohlenstoffatomen oder durch zweifach verknüpftes Dioxyalkylen mit 1 bis 4 Kohlenstoffatomen, wobei die beiden letztgenannten Reste selbst einfach oder mehrfach, gleichartig oder verschieden substituiert sein
können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen,

$R^2$ für Cycloalkyl mit 3 bis 7 Kohlenstoffatomen steht, wobei diese Reste einfach bis vierfach, gleichartig oder verschieden substituiert sein können durch Halogen und/oder Alkyl mit 1 bis 4 Kohlenstoffatomen, oder

$R^2$ für Cycloalkenyl mit 3 bis 7 Kohlenstoffatomen steht, wobei diese Reste einfach bis vierfach, gleichartig oder verschieden substituiert sein können durch Halogen und/oder Alkyl mit 1 bis 4 Kohlenstoffatomen, oder

$R^2$ für gesättigtes oder ungesättigtes, gegebenenfalls benzokondensiertes Heterocyclyl mit 3 bis 7 Ringgliedern im Heterocyclus steht, von denen jeweils 1 bis 3 gleiche oder verschiedene Heteroatome, wie Sauerstoff, Stickstoff oder Schwefel, sind, wobei die Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen und/oder Halogenalkyl mit 1 bis 4 Kohlenstoffatomen und 1 bis 5 gleichen oder verschiedenen Halogenatomen, oder

$R^2$ für Aryl mit 6 bis 10 Kohlenstoffatomen steht, wobei jeder dieser Reste einfach bis fünffach, gleichartig oder verschieden substituiert sein kann durch

Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Phenyl, Phenoxy, Phenylthio, Benzyloxy, Benzylthio oder Phenylethyloxy,
oder durch zweifach verknüpftes Alkylen mit 3 oder 4 Kohlenstoffatomen oder durch zweifach verknüpftes Dioxyalkylen mit 1 oder 2 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen,
und

n für die Zahlen 0, 1, 2 oder 3 steht,

erhält, indem man racemische N-Acylamine der Formel

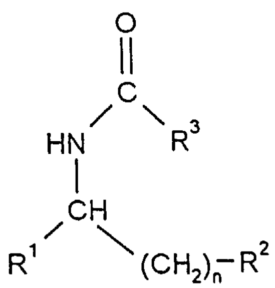

(II),

in welcher

$R^1$, $R^2$ und n die oben angegebenen Bedeutungen haben und

$R^3$ für Wasserstoff, Amino, Dialkylamino mit 1 bis 6 Kohlenstoffatomen in jedem Alkylteil, Alkylthio mit 1 bis 6 Kohlenstoffatomen, geradkettiges oder verzweigtes Alkyl mit 1 bis 10 Kohlenstoffatomen oder geradkettiges oder verzweigtes Alkoxy mit 1 bis 6 Kohlenstoffatomen steht, wobei die Alkylreste oder Alkoxyreste einfach oder mehrfach, gleichartig oder verschieden substituiert sein können durch Halogen, Cyano, Nitro, Amino, Hydroxy, Formyl, Carboxy, Carbamoyl;
jeweils geradkettiges oder verzweigtes Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
oder durch zweifach verknüpftes Alkylen mit 1 bis 6 Kohlenstoffatomen oder durch zweifach verknüpftes Dioxyalkylen mit 1 bis 4 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9, gleichen oder verschiedenen Halogenatomen, oder durch Phenyl, oder

$R^3$ für Cycloalkyl mit 3 bis 7 Kohlenstoffatomen steht, wobei diese Reste einfach bis vierfach, gleichartig oder verschieden substituiert sein können durch Halogen und/oder Alkyl mit 1 bis 4 Kohlenstoffatomen, oder

$R^3$ für Cycloalkenyl mit 3 bis 7 Kohlenstoffatomen steht, wobei diese Reste einfach bis vierfach, gleichartig oder verschieden substituiert sein können durch Halogen und/oder Alkyl mit 1 bis 4 Kohlenstoffatomen, oder

$R^3$ für gesättigtes oder ungesättigtes Heterocyclyl mit 3 bis 7 Ringgliedern steht, von denen jeweils 1 bis 3 gleiche oder verschiedene Heteroatome, wie Sauerstoff, Stickstoff oder Schwefel, sind, wobei die Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen und/oder Halogenalkyl mit 1 bis 4 Kohlenstoffatomen und 1 bis 5 gleichen oder verschiedenen Halogenatomen, oder

$R^3$ für Aryl mit 6 bis 10 Kohlenstoffatomen steht, wobei jeder dieser Reste einfach bis fünffach, gleichartig oder verschieden substituiert sein kann durch Halogen, Cyano, geradkettiges oder verzweigtes Alkyl mit 1 bis 4 Kohlenstoffatomen,
geradkettiges oder verzweigtes Halogenalkyl mit 1 bis 4 Kohlen stoffatomen und 1 bis 9 gleichen oder verschiedenen Halogenatomen,
geradkettiges oder verzweigtes A'k°Xy oder Alkylthio mit 1 bis 4 Kohlenstoffatomen,
geradkettiges oder verzweigtes Halogenalkoxy oder Halogenalkylthio mit 1 bis 4 Kohlenstoffatomen und 1 bis 9 gleichen öder verschiedenen Halogenatomen,
Phenyl, Phenoxy, Phenylthio, Benzyloxy, Benzylthio oder Phenylethyloxy,
oder durch zweifach verknüpftes Alkylen mit 3 oder 4 Kohlenstoffatomen oder durch zweifach verknüpftes Dioxyalkylen mit 1 oder 2 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen,

mit Lipase aus Candida antarctica, Lipase aus Rhizopus niveus, Lipase aus Pseudomonas Sp. oder Lipase aus Mucor javanicus in Gegenwart von Wasser sowie gegebenenfalls in Gegenwart eines organischen Verdünnungsmittels bei einem pH - Wert zwischen 3,0 und 10,0 bei Temperaturen zwischen 0° C und 80 C ° umsetzt.

Unter (R)-Aminen sind diejenigen optisch aktiven Verbindungen der Formel (I) zu verstehen, die am asymmetrisch substituierten Kohlenstoffatom die (R)-Konfiguration aufweisen. In der Formel (I) ist das asymmetrisch substituierte Kohlenstoffatom durch (*) gekennzeichnet.

Es ist als äußerst überraschend zu bezeichnen, daß sich nach dem erfindungsgemäßen Verfahren (R)-Amine herstellen lassen, denn aus dem Stand der Technik war bisher nur bekannt, daß (S)-Amine mit Hilfe von Biokatalysatoren aus Racematen von N-Acylaminen zugänglich sind.

Das erfindungsgemäße Verfahren zeichnet sich durch eine Reihe von Vorteilen aus. So ermöglicht es die Herstellung von (R)-Aminen der Formel (I) in extrem hoher optischer Reinheit. Günstig ist auch, daß die als Ausgangsprodukte benötigten N-Acylamine in einfacher Weise zugänglich sind und der Acyl-Rest breit variierbar ist. Schließlich bereitet auch die Durchführung der Umsetzung und die Isolierung der gewünschten Substanzen keinerlei Schwierigkeiten.

Verwendet man racemisches N-[1-(4-Chlorphenyl)-ethyl]-acetamid als Ausgangssubstanz und Lipase aus Candida antarctica als Biokatalysator, so kann der Verlauf des erfindungsgemäßen Verfahrens durch das folgende Formelschema veranschaulicht werden.

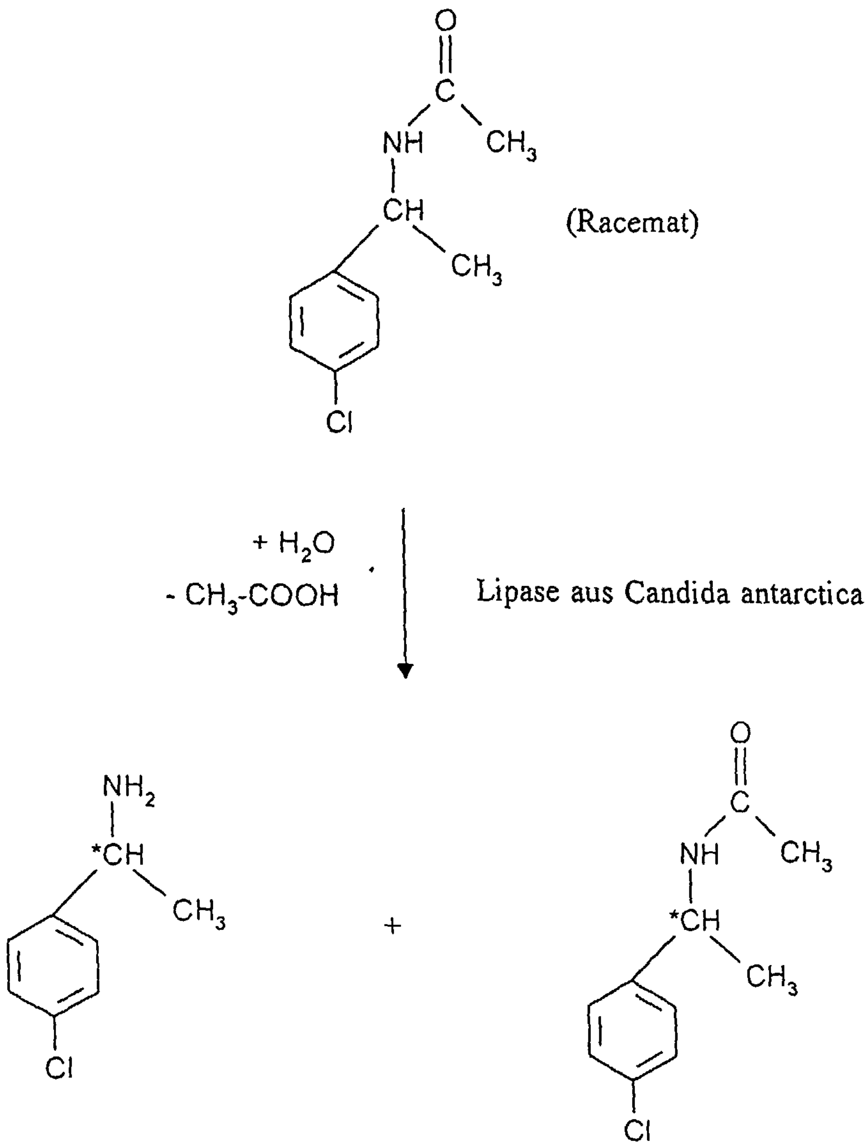


(R)-Amin                (S)-Acyl-amin

Die bei der Durchführung des erfindungsgemäßen Verfahrens als Ausgangsstoffe benötigten racemischen N-Acylamine sind durch die Formel (II) allgemein definiert.

$R^1$ steht bevorzugt für Methyl, Ethyl oder n-Propyl.

$R^2$ steht bevorzugt für Cyclohexyl, Norbornyl oder Cyclohexenyl, wobei diese Reste einfach bis vierfach, gleichartig oder verschieden substituiert sein können durch Fluor, Chlor, Methyl und/oder Ethyl, oder für Furyl, Pyridyl, Thienyl, Benzofuryl, Chinolyl oder Benzothienyl, die einfach bis dreifach, gleichartig oder verschieden substituiert sein können durch Fluor, Chlor, Brom, Methyl, Ethyl, n-Propyl, i-Propyl, n-Butyl, i-Butyl, s-Butyl, t-Butyl, Methoxy, Ethoxy, n-Propoxy, i-Propoxy, Trifluormethyl und/oder Trifluorethyl, oder

$R^2$ steht bevorzugt für Phenyl oder Naphthyl, wobei jeder dieser Reste einfach bis fünffach, gleichartig oder verschieden substituiert sein kann durch Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder

i-Propylamino, Dimethylamino, Diethylamino, Methoxycarbonyl, Ethoxycarbonyl, Phenyl, Phenoxy, Phenylthio, Benzyloxy, Benzylthio oder Phenylethyloxy, oder durch jeweils gegebenenfalls einfach oder mehrfach, gleich oder verschieden durch Fluor, Chlor, Methyl, Trifluormethyl, Ethyl, n- oder iPropyl substituiertes, jeweils zweifach verknüpftes Trimethylen (Propan-1,3-diyl), Methylendioxy oder Ethylendioxy.

n steht auch bevorzugt für die Zahlen 0, 1, 2 oder 3.

$R^3$ steht bevorzugt für Wasserstoff, Amino, Dimethylamino, Methylthio, Methyl, Ethyl, n- oder i-Propyl, n-, i-, s-Butyl, Pentyl, Hexyl, Heptyl, Octyl, Methoxy, Ethoxy, Methoxymethyl, Hydroxymethyl, 1-Hydroxy-1-ethyl, 2-Hydroxycarbonyl-1-hydroxy-1-ethyl, 2-Hydroxycarbonyl-1-ethyl, 3-Hydroxycarbonyl-1-propyl und Benzyl.

Die racemischen N-Acylamine der Formel (II) sind bekannt oder lassen sich nach bekannten Methoden durch Acylierung von Aminen herstellen. So erhält man N-Acylamine der Formel (II) zum Beispiel dadurch, daß man racemische Amine mit Säurechloriden oder Säureanhydriden umsetzt.

Die als Biokatalysatoren einsetzbaren Lipasen sind bekannt.

Die Lipasen können bei der Durchführung des erfindungsgemäßen Verfahrens entweder nativ oder in modifizierter Form, zum Beispiel mikroverkapselt oder an anorganische oder organische Trägermaterialien gebunden, eingesetzt werden. Als Trägermaterialien kommen dabei zum Beispiel Celite, Zeolithe, Polysaccharide, Polyamide und Polystyrolharze in Frage.

Als organische Verdünnungsmittel, die bei der Durchführung des erfindungsgemäßen Verfahrens einsetzbar sind, kommen alle für derartige Reaktionen üblichen Solventien in Betracht. Vorzugsweise verwendbar sind Alkohole, wie Methanol, Ethanol, n-Butanol, Benzylalkohol und Phenethylalkohol, ferner Ether, wie Tetrahydrofuran und Dioxan, außerdem Kohlenwasserstoffe, wie Pentan und Hexan, weiterhin Amide, wie Dimethylformamid, oder auch stark polare Lösungsmittel, wie Dimethylsulfoxid. Als organische Verdünnungsmittel kommen schließlich auch Emulgatoren und oberflächenaktive Substanzen in Frage, die als Phasentransfer-Katalysatoren fungieren können. Vorzugsweise in Betracht kommen Alkylaryl-polyglykol-ether, Polyethylenoxid-fettsäure-ester, Polyethylenoxid-fettalkoholether und Ethoxylate des 4-(1,1,3,3-Tetramethylbutyl)-phenols.

Zur Einstellung des gewünschten pH-Wertes kommen bei der Durchführung des erfindungsgemäßen Verfahrens alle üblichen Puffersysteme in Betracht. Vorzugsweise verwendbar sind Natriumdihydrogenphosphat/Dinatriumhydrogenphosphat-Gemische, Citrat-Puffer, Glycin-Puffer und andere Gemische aus geeigneten Säuren und Basen.

Der pH-Wert kann bei der Durchführung des erfindungsgemäßen Verfahrens innerhalb eines bestimmten Bereiches variiert werden. Im allgemeinen arbeitet man bei pH-Werten zwischen 3,0 und 10,0, vorzugsweise zwischen 4,0 und 9,0.

Die Reaktionstemperaturen können bei der Durchführung des erfindungsgemäßen Verfahrens innerhalb eines bestimmten Bereiches variiert werden. Im allgemeinen arbeitet man bei Temperaturen zwischen 0°C und 80°C, vorzugsweise zwischen 20°C und 70°C.

Die Konzentrationen an racemischen N-Acyl-aminen der Formel (II) können bei der Durchführung des erfindungsgemäßen Verfahrens innerhalb eines bestimmten Bereiches variiert werden. Im allgemeinen verwendet man Reaktionsgemische, in denen die Konzentration an racemischem N-Acylamin der Formel (II) zwischen 1 und 200 g/ Liter, vorzugsweise zwischen 2 und 100 g / Liter beträgt.

Bei der Durchführung des erfindungsgemäßen Verfahrens setzt man auf 1 g an racemischem N-Acyl-amin der Formel (II) im allgemeinen 0,01 bis 200 g, vorzugsweise 0,05 bis 100 g an Biokatalysator ein. Die Aufarbeitung erfolgt nach üblichen Methoden. Im allgemeinen geht man so vor, daß man den Biokatalysator abtrennt und die gewünschten Komponenten aus dem verbleibenden Reaktionsgemisch durch Destillation, fraktionierte Kristallisation, Säure-Base-Lösungsmittel-Extraktion oder auf anderem Wege isoliert. Dabei fallen die (R)-Amine der Formel (I) entweder in freiem Zustand oder in Form von Salzen an, aus denen die (R)-Amine durch Behandlung mit Base freigesetzt werden können. Außerdem lassen sich aus dem Reaktionsgemisch auch die (S)-Enantiomeren der N-Acyl-amine der Formel (II) abtrennen. Letztere können durch einen weiteren chemischen Reaktionsschritt, zum Beispiel saure oder basische Verseifung, oder auf enzymatischem Wege in die freien (S)-Amine oder deren Säureadditions-Salze überführt werden. Die nach dem erfindungsgemäßen Verfahren herstellbaren (R)-Amine der Formel (I) sind wertvolle Zwischenprodukte zur Herstellung von Pharmazeutika oder von Wirkstoffen mit insektiziden, fungiziden oder herbiziden Eigenschaften (vgl. EP-A 0 519 211, EP-A 0 453 137, EP-A 0 283 879, EP-A 0 264 217 und EP-A 0 341 475). So erhält man zum Beispiel die fungizid wirksame Verbindung der Formel

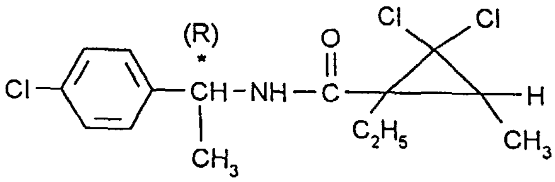

(III)

indem man das (R)-1-(4-Chlor-phenyl)-ethylamin der Formel

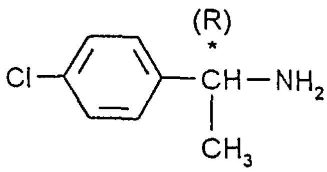

(I-1)

mit 2,2-Dichlor-1-ethyl-3-methyl-1-cyclopropancarbonsäurechlorid der Formel

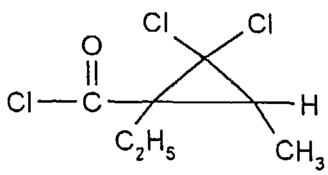

(IV)

in Gegenwart eines Säurebindemittels und in Gegenwart eines inerten organischen Verdünnungsmittels umsetzt.

Die Durchführung des erfindungsgemäßen Verfahrens wird durch die folgenden Beispiele veranschaulicht.

## Herstellungsbeispiele

### Beispiel 1

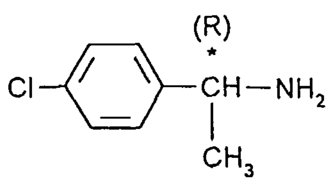

Ein Gemisch aus 50 mg racemischem N-[1-(4-Chlor-phenyl)-ethyl]-acetamid und 100 mg Lipase aus Candida antarctica (Novozym 435®; E.C. Nr. 3.1.1.3) wurde mit einer 50 mM wäßrigen Lösung eines Natriumdihydrogenphosphat/Dinatriumhydrogenphosphat-Puffergemisches auf ein Volumen von 10 ml aufgefüllt. Das Reaktionsgemisch, das einen pH-Wert von 8 aufwies, wurde 168 Stunden bei 50°C geschüttelt. Danach wurde 5 Minuten zentrifugiert und dann die flüssige Phase analysiert. Dazu wurde dem Ansatz ein definierter Anteil entnommen, der bei -60°C eine Stunde lang eingefroren und anschließend gefriergetrocknet wurde. Die trockene Probe wurde mit Methanol unter Zugabe von Molekularsieb eine Stunde lang unter mehrmaliger intensiver Behandlung mit einem Vortex-Schüttler extrahiert. Die nach dem Zentrifugieren erhaltene wasserfreie, methanolische Phase wurde gaschromatographisch analysiert. Zur Bestimmung der Konzentrationen an racemischem Amin und an racemischem N-Acetyl-amin bei der Ermittlung des Umsatzes wurde eine konventionelle Reversed Phase RP 18-Säule in der HPLC-Analytik verwendet.

Als Laufmittel wurde ein Gemisch aus Acetonitril / 20 mM Phosphatpuffer, pH 2,0, 80:20 (v/v) benutzt. Zur UV-Detektion wurde eine Wellenlänge von 220 nm verwendet. Sowohl für das racemische Amin als auch für das N-Acetyl-Amin wurde eine Kalibrierung nach der Methode des externen Standards durchgeführt. Zur chiralen Analytik wurde die Kapillargaschromatographie eingesetzt. Als Säule diente eine Kapillar-Glassäule mit einer Länge von 20 m. Als Trennmaterial wurde eine chirale Mischphase verwendet, als Trägergas diente $H_2$ mit einem Trägergasdruck von 0,4 bar. Es wurde ein Temperaturgradient von 80°C // 1min iso // 2,5°C/min // 150°C // 10°C/min // 200°C verwendet.

Zur Beurteilung der Enantioselektivität der Umsetzung wurde der Enantiomerenüberschuß (ee-Wert) herangezogen, der sich wie folgt berechnet:

$$ee = \frac{(R - S)}{(R + S)} \times 100\ \%$$

Dabei bezeichnen R und S die Konzentration der einzelnen Enantiomere des gebildeten Amins.

Auf diese Weise ergibt sich, daß das (R)-1-(4-Chlor-phenyl)-ethylamin in einer Ausbeute von 43 % mit einem ee-Wert von >99 % entstanden ist.

## Beispiel 2

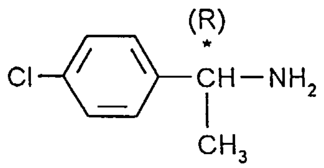

Ein Gemisch aus 3 mg racemischem N-[1-(4-Chlor-phenyl)ethyl]-acetamid und 0,75 mg aufgereinigte freie Lipase B aus Candida antarctica wurde mit einer 50 mM wäßrigen Lösung eines Natriumdihydrogenphosphat/Dinatriumhydrogenphosphat-Puffergemisches auf ein Volumen von 1,5 ml aufgefüllt. Das Reaktionsgemisch, das einen pH-Wert von 8 aufwies, wurde 360 Stunden bei 30°C geschüttelt. Danach wurde in der im Beispiel 1 angegebenen Weise aufgearbeitet und analysiert. Dadurch ergibt sich, daß das (R)-1-(4-Chlorphenyl)-ethylamin in einer Ausbeute von 25 % mit einem ee-Wert von > 99 % entstanden ist.

## Beispiel 3

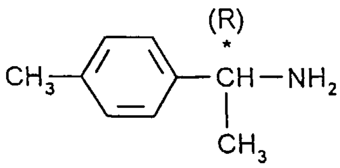

Ein Gemisch aus 1,3 mg racemischem N-[1-(4-Methylphenyl)-ethyl]-acetamid und 15 mg Lipase aus Candida antarctica (Novozym 435 ®; E.C.Nr. 3.1.1.3) wurde mit einer 50 mM wäßrigen Lösung eines Natriumdihydrogenphosphat/Dinatriumhydrogenphosphat-Puffergemisches auf ein Volumen von 1,5 ml aufgefüllt. Das Reaktionsgemisch, das einen pH-Wert von 8 aufwies, wurde 400 Stunden bei 50°C geschüttelt. Danach wurde in der im Beispiel 1 angegebenen Weise aufgearbeitet und analysiert. Dadurch ergibt sich, daß das (R)-1-(4-Methylphenyl)-ethylamin in einer Ausbeute von 32 % mit einem ee-Wert von > 90 % entstanden ist.

**Beispiel 4**

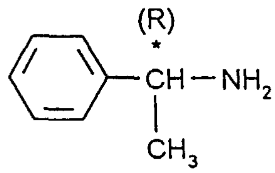

Ein Gemisch aus 1,2 mg racemischem N-(1-Phenyl-ethyl)-acetamid und 15 mg Lipase aus Candida antarctica (Novozym 435 ®; E.C.Nr. 3.1.1.3) wurde mit einer 50 mM wäßrigen Lösung eines Natriumdihydrogenphosphat/Dinatriumhydrogenphosphat-Puffergemisches auf ein Volumen von 1,5 ml aufgefüllt. Das Reaktionsgemisch, das einen pH-Wert von 8 aufwies, wurde 400 Stunden bei 50°C geschüttelt. Danach wurde in der im Beispiel 1 angegebenen Weise aufgearbeitet und analysiert. Dadurch ergibt sich, daß das (R)-1-Phenyl-ethylamin in einer Ausbeute von 7,6 % mit einem ee-Wert von > 90 % entstanden ist.

**Beispiel 5**

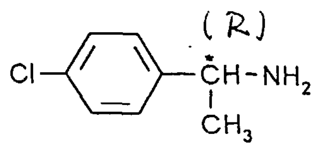

Ein Gemisch aus 200 mg (0,88 mmol) racemischem N-[1-(4-Chlor-phenyl)-ethyl]methoxyacetamid und 50 U Lipase aus Candida antarctica (Novozym 435®) wurde mit einer 50 mM wäßrigen Lösung eines Natriumdihydrogenphosphat/Dinatriumhydrogenphosphat-Puffergemisches auf ein Volumen von 7 ml aufgefüllt. Das Reaktionsgemisch, das einen pH-Wert von 8 aufwies, wurde 48 Stunden bei 40°C gerührt. Danach wurde das Reaktionsgemisch dreimal mit Diisopropylether extrahiert. Die vereinigten organischen Phasen wurden unter vermindertem Druck eingeengt. Der verbleibende Rückstand wurde mit Hilfe einer chiralen Säule gaschromatisch untersucht. Dadurch ergibt sich, daß die Reaktion mit einem Umsatz von 48 % verlaufen ist. Das erhaltene (R)-1-(4-Chlorphenyl)ethylamin weist einen ee-Wert von 99 % auf.

Für das (S)-N-[1-(4-Chlor-phenyl)-ethyl]-methoxyacetamid ergibt sich ein ee-Wert von 92 %.

## Patentansprüche

1. Verfahren zur Herstellung von (R)-Aminen der Formel

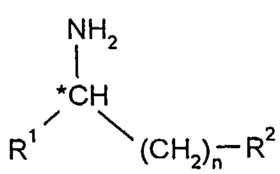

(I),

in welcher

$R^1$ für geradkettiges oder verzweigtes Alkyl mit 1 bis 8 Kohlenstoffatomen steht, wobei die Alkylreste einfach oder mehrfach, gleichartig oder verschieden substituiert sein können durch
Halogen, Cyano, Amino, Hydroxy, Formyl, Carboxy, Carbamoyl, jeweils geradkettiges oder verzweigtes Alkoxy oder Alkylthio mit jeweils 1 bis 6 Kohlenstoffatomen;
jeweils geradkettiges oder verzweigtes Halogenalkoxy oder Halogenalkylthio mit jeweils 1 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
oder durch zweifach verknüpftes Alkylen mit 1 bis 6 Kohlenstoffatomen oder durch zweifach verknüpftes Dioxyalkylen mit 1 bis 4 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen,

$R^2$ für Cycloalkyl mit 3 bis 7 Kohlenstoffatomen steht, wobei diese Reste einfach bis vierfach, gleichartig oder verschieden substituiert sein können durch Halogen und/oder Alkyl mit 1 bis 4 Kohlenstoffatomen, oder

$R^2$ für Cycloalkenyl mit 3 bis 7 Kohlenstoffatomen steht, wobei diese Reste einfach bis vierfach, gleichartig oder verschieden substituiert sein können durch Halogen und/oder Alkyl mit 1 bis 4 Kohlenstoffatomen, oder

$R^2$ für gesättigtes oder ungesättigtes, gegebenenfalls benzokondensiertes Heterocyclyl mit 3 bis 7 Ringgliedern im Heterocyclus steht, von denen jeweils 1 bis 3 gleiche oder verschiedene Heteroatome, wie Sauerstoff, Stickstoff oder Schwefel, sind, wobei die Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen und/oder Halogenalkyl mit 1 bis 4 Kohlenstoffatomen und 1 bis 5 gleichen oder verschiedenen Halogenatomen, oder

$R^2$ für Aryl mit 6 bis 10 Kohlenstoffatomen steht, wobei jeder dieser Reste einfach bis fünffach, gleichartig oder verschieden substituiert sein kann durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Phenyl, Phenoxy, Phenylthio, Benzyloxy, Benzylthio oder Phenylethyloxy,
oder durch zweifach verknüpftes Alkylen mit 3 oder 4 Kohlenstoffatomen oder durch zweifach verknüpftes Dioxyalkylen mit 1 oder 2 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen,
und

n für die Zahlen 0, 1, 2 oder 3 steht,

dadurch gekennzeichnet, daß man racemische N-Acylamine der Formel

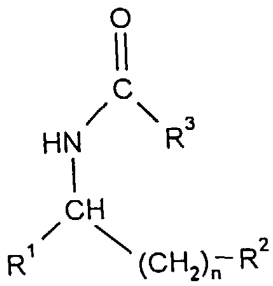

(II),

in welcher

$R^1$, $R^2$ und n die oben angegebenen Bedeutungen haben und

$R^3$ für Wasserstoff, Amino, Dialkylamino mit 1 bis 6 Kohlenstoffatomen in jedem Alkylteil, Alkylthio mit 1 bis 6 Kohlenstoffatomen, geradkettiges oder verzweigtes Alkyl mit 1 bis 10 Kohlenstoffatomen oder geradkettiges oder verzweigtes Alkoxy mit 1 bis 6 Kohlenstoffatomen steht, wobei die Alkylreste oder Alkoxyreste einfach oder mehrfach, gleichartig oder verschieden substituiert sein können durch Halogen, Cyano, Nitro, Amino, Hydroxy, Formyl, Carboxy, Carbamoyl;
jeweils geradkettiges oder verzweigtes Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
oder durch zweifach verknüpftes Alkylen mit 1 bis 6 Kohlenstoffatomen oder durch zweifach verknüpftes Dioxyalkylen mit 1 bis 4 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9, gleichen oder verschiedenen Halogenatomen, oder
durch Phenyl, oder

$R^3$ für Cycloalkyl mit 3 bis 7 Kohlenstoffatomen steht, wobei diese Reste einfach bis vierfach, gleichartig oder verschieden substituiert sein können durch Halogen und/oder Alkyl mit 1 bis 4 Kohlenstoffatomen, oder

$R^3$ für Cycloalkenyl mit 3 bis 7 Kohlenstoffatomen steht, wobei diese Reste einfach bis vierfach, gleichartig oder verschieden substituiert sein können durch Halogen und/oder Alkyl mit 1 bis 4 Kohlenstoffatomen, oder

$R^3$ für gesättigtes oder ungesättigtes Heterocyclyl mit 3 bis 7 Ringgliedern steht, von denen jeweils 1 bis 3 gleiche oder verschiedene Heteroatome, wie Sauerstoff, Stickstoff oder Schwefel, sind, wobei die Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen und/oder Halogenalkyl mit 1 bis 4 Kohlenstoffatomen und 1 bis 5 gleichen oder verschiedenen Halogenatomen, oder

$R^3$ für Aryl mit 6 bis 10 Kohlenstoffatomen steht, wobei jeder dieser Reste einfach bis fünffach, gleichartig oder verschieden substituiert sein kann durch Halogen, Cyano, geradkettiges oder verzweigtes Alkyl mit 1 bis 4 Kohlenstoffatomen,
geradkettiges oder verzweigtes Halogenalkyl mit 1 bis 4 Kohlenstoffatomen und 1 bis 9 gleichen oder verschiedenen Halogenatomen,

geradkettiges oder verzweigtes Alkoxy oder Alkylthio mit 1 bis 4 Kohlenstoffatomen,
geradkettiges oder verzweigtes Halogenalkoxy oder Halogenalkylthio mit 1 bis 4 Kohlenstoffatomen und 1 bis 9 gleichen oder verschiedenen Halogenatomen,
Phenyl, Phenoxy, Phenylthio, Benzyloxy, Benzylthio oder Phenylethyloxy,
oder durch zweifach verknüpftes Alkylen mit 3 oder 4 Kohlenstoffatomen oder durch zweifach verknüpftes Dioxyalkylen mit 1 oder 2 Kohlenstoffatomen, wobei die beiden letztgenannten Reste selbst einfach oder mehrfach, gleichartig oder verschieden substituiert sein können durch Halogen, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen,

mit Lipase aus Candida antarctica, Lipase aus Rhizopus niveus, Lipase aus Pseudomonas Sp. oder Lipase aus Mucor javanicus in Gegenwart von Wasser sowie gegebenenfalls in Gegenwart eines organischen Verdünnungsmittels bei einem pH - Wert zwischen 3,0 und 10,0 bei Temperaturen zwischen 0° C und 80 C ° umsetzt.

2. Verfahren gemäß Anspruch 1, dadurch gekennzeichnet, daß man als racemisches N-Acylamin die Verbindung der Formel

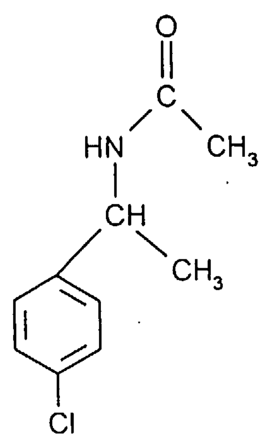

einsetzt.

3. Verfahren gemäß Anspruch 1, dadurch gekennzeichnet, daß man bei einem pH-Wert zwischen 4,0 und 9,0 arbeitet.

4. Verfahren gemäß Anspruch 1, dadurch gekennzeichnet, daß man bei Temperaturen zwischen 20°C und 70°C arbeitet.

## Claims

1. Process for preparing (R)-amines of the formula

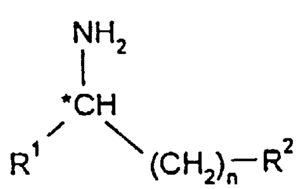

(I),

in which

$R^1$ represents straight-chain or branched alkyl having from 1 to 8 carbon atoms, where the alkyl radicals can be

substituted identically or differently, once or more than once, by halogen, cyano, amino, hydroxyl, formyl, carboxyl, carbamoyl, in each case straight-chain or branched alkoxy or alkylthio having in each case from 1 to 6 carbon atoms;
in each case straight-chain or branched halogenoalkoxy or halogenoalkylthio having in each case from 1 to 6 carbon atoms and from 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroxyiminoalkyl or alkoxyiminoalkyl having in each case from 1 to 6 carbon atoms in the individual alkyl moieties;
or by doubly linked alkylene having from 1 to 6 carbon atoms or by doubly linked dioxyalkylene having from 1 to 4 carbon atoms, where the two latter radicals can themselves be substituted identically or differently, once or more than once, by halogen, straight-chain or branched alkyl having from 1 to 4 carbon atoms and/or straight-chain or branched halogenoalkyl having from 1 to 4 carbon atoms and from 1 to 9 identical or different halogen atoms,

$R^2$ represents cycloalkyl having from 3 to 7 carbon atoms, where these radicals can be substituted identically or differently, once to four times, by halogen and/or alkyl having from 1 to 4 carbon atoms, or

$R^2$ represents cycloalkenyl having from 3 to 7 carbon atoms, where these radicals can be substituted identically or differently, once to four times, by halogen and/or alkyl having from 1 to 4 carbon atoms, or

$R^2$ represents saturated or unsaturated, optionally benzo-fused heterocyclyl having from 3 to 7 ring members in the heterocycle, of which in each case from 1 to 3 are identical or different heteroatoms, such as oxygen, nitrogen or sulphur, where the radicals can be substituted identically or differently, once to three times, by halogen, alkyl having from 1 to 4 carbon atoms, alkoxy having from 1 to 4 carbon atoms and/or halogenoalkyl having from 1 to 4 carbon atoms and from 1 to 5 identical or different halogen atoms, or

$R^2$ represents aryl having from 6 to 10 carbon atoms, where each of these radicals can be substituted identically or differently, once to five times, by
halogen, cyano, nitro, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case from 1 to 6 carbon atoms;
in each case straight-chain or branched alkenyl or alkenyloxy having in each case from 2 to 6 carbon atoms;
in each case straight-chain or branched halogenoalkyl, halogenoalkoxy, halogenoalkylthio, halogenoalkylsulphinyl or halogenoalkylsulphonyl having in each case from 1 to 6 carbon atoms and from 1 to 13 identical or different halogen atoms;
in each case straight-chain or branched halogenoalkenyl or halogenoalkenyloxy having in each case from 2 to 6 carbon atoms and from 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroxyiminoalkyl or alkoxyiminoalkyl having in each case from 1 to 6 carbon atoms in the individual alkyl moieties;
phenyl, phenoxy, phenylthio, benzyloxy, benzylthio or phenylethyloxy,
or by doubly linked alkylene having 3 or 4 carbon atoms or by doubly linked dioxyalkylene having 1 or 2 carbon atoms, where the two latter radicals can themselves be substituted identically or differently, once or more than once, by halogen, straight-chain or branched alkyl having from 1 to 4 carbon atoms and/or straight-chain or branched halogenoalkyl having from 1 to 4 carbon atoms and from 1 to 9 identical or different halogen atoms, and

n represents the numbers 0, 1, 2 or 3, characterized in that racemic N-acylamines of the formula

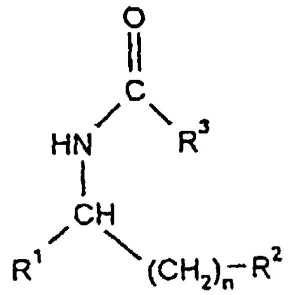

(II),

in which
$R^1$, $R^2$ and n have the abovementioned meanings, and

$R^3$ represents hydrogen, amino, dialkylamino having from 1 to 6 carbon atoms in each alkyl moiety, alkylthio having from 1 to 6 carbon atoms, straight-chain or branched alkyl having from 1 to 10 carbon atoms or straight-chain or branched alkoxy having from 1 to 6 carbon atoms, where the alkyl radicals or alkoxy radicals can be substituted identically or differently, once or more than once, by halogen, cyano, nitro, amino, hydroxyl, formyl, carboxyl, carbamoyl;
in each case straight-chain or branched alkoxy, alkylthio, alkylsulphinyl or alkylsulphonyl having in each case from 1 to 6 carbon atoms;
in each case straight-chain or branched alkenyl or alkenyloxy having in each case from 2 to 6 carbon atoms;
in each case straight-chain or branched halogenoalkoxy, halogenoalkylthio, halogenoalkylsulphinyl or halogenoalkylsulphonyl having in each case from 1 to 6 carbon atoms and from 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroxyiminoalkyl or alkoxyiminoalkyl having in each case from 1 to 6 carbon atoms in the individual alkyl moieties;
or by doubly linked alkylene having from 1 to 6 carbon atoms or by doubly linked dioxyalkylene having from 1 to 4 carbon atoms, where the two latter radicals can themselves be substituted identically or differently, once or more than once, by halogen, straight-chain or branched alkyl having from 1 to 4 carbon atoms and/or straight-chain or branched halogenoalkyl having from 1 to 4 carbon atoms and from 1 to 9 identical or different halogen atoms, or
by phenyl, or

$R^3$ represents cycloalkyl having from 3 to 7 carbon atoms, where these radicals can be substituted identically or differently, once to four times, by halogen and/or alkyl having from 1 to 4 carbon atoms, or

$R^3$ represents cycloalkenyl having from 3 to 7 carbon atoms, where these radicals can be substituted identically or differently, once to four times, by halogen and/or alkyl having from 1 to 4 carbon atoms, or

$R^3$ represents saturated or unsaturated heterocyclyl having from 3 to 7 ring members, of which in each case from 1 to 3 are identical or different heteroatoms, such as oxygen, nitrogen or sulphur, where the radicals can be substituted identically or differently, once to three times, by halogen, alkyl having from 1 to 4 carbon atoms, alkoxy having from 1 to 4 carbon atoms and/or halogenoalkyl having from 1 to 4 carbon atoms and from 1 to 5 identical or different halogen atoms, or

$R^3$ represents aryl having from 6 to 10 carbon atoms, where each of these radicals can be substituted identically or differently, once to five times, by halogen, cyano, straight-chain or branched alkyl having from 1 to 4 carbon atoms,
straight-chain or branched halogenoalkyl having from 1 to 4 carbon atoms and from 1 to 9 identical or different halogen atoms,
straight-chain or branched alkoxy or alkylthio having from 1 to 4 carbon atoms,
straight-chain or branched halogenoalkoxy or halogenoalkylthio having from 1 to 4 carbon atoms and from 1 to 9 identical or different halogen atoms,
phenyl, phenoxy, phenylthio, benzyloxy, benzylthio or phenylethyloxy,
or by doubly linked alkylene having 3 or 4 carbon atoms or by doubly linked dioxyalkylene having 1 or 2 carbon

atoms, where the two latter radicals can themselves be substituted identically or differently, once or more than once, by halogen, straight-chain or branched alkyl having from 1 to 4 carbon atoms and/or straight-chain or branched halogenoalkyl having from 1 to 4 carbon atoms and from 1 to 9 identical or different halogen atoms, are reacted with lipase from Candida antarctica, lipase from Rhizopus nivens, lipase from Pseudomonas sp. Or lipase from Mucor javanicus, in the presence of water and optionally in the presence of an organic diluent, at a pH of between 3.0 and 10.0 and at temperatures of between 0°C and 80°C.

**2.** Process according to Claim 1, characterized in that the compound of the formula

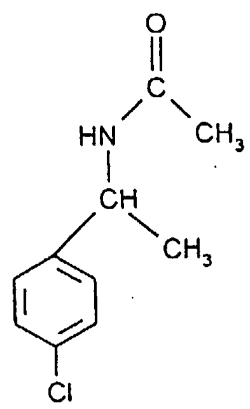

is employed as the racemic N-acylamine.

**3.** Process according to Claim 1, characterized in that it is carried out at a pH of between 4.0 and 9.0.

**4.** Process according to Claim 1, characterized in that it is carried out at temperatures of between 20°C and 70°C.

**Revendications**

**1.** Procédé de préparation d'amines R de formule :

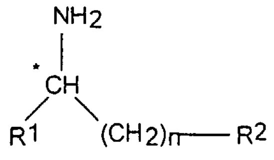

(I)

dans laquelle :

$R^1$ représente un radial alcoyle linéaire ou ramifié ayant 1 à 8 atomes de carbone, où le reste alcoyle peut être substitué une ou plusieurs fois, de manière identique ou différente, par
un atome d'halogène, un radical cyano, amino, hydroxyle, formyle, carboxyle, carbamoyle, alcoxy ou alcoylthio chaque fois linéaire ou ramifié ayant chaque fois 1 à 6 atomes de carbone ;
un radical halogénoalcoxy ou halogénoalcoylthio, chaque fois linéaire ou ramifié, ayant chaque fois 1 à 6 atomes de carbone et 1 à 13 atomes d'halogène identiques ou différents ;
un radical alcoylamino, dialcoylamino, alcoylcarbonyle, alcoylcarbonyloxy, alcoxycarbonyle, alcoylsulfonyloxy, hydroximinialcoyle ou alcoximinoalcoyle chaque fois linéaire ou ramifié ayant chaque fois 1 à 6 atomes de carbone dans les unités alcoyle individuelles ;
ou par un radical alcoylène relié deux fois, ayant 1 à 6 atomes de carbone ou par un radical dioxyalcoylène relié deux fois, ayant 1 à 4 atomes de carbone, où les deux restes cités en dernier lieu peuvent être eux-mêmes substitués une ou plusieurs fois, de manière identique ou différente par un atome d'halogène, un radical alcoyle linéaire ou ramifié ayant 1 à 4 atomes de carbone et/ou un radical halogénoalcoyle linéaire ou

ramifié ayant 1 à 4 atomes de carbone et 1 à 9 atomes d'halogène identiques ou différents,
$R^2$ représente un radical cycloalcoyle ayant 3 à 7 atomes de carbone, où ce reste peut être substitué une à quatre fois, de manière identique ou différente, par un atome d'halogène et/ou un radical alcoyle ayant 1 à 4 atomes de carbone, ou
$R^2$ représente un radical cycloalcényle ayant 3 à 7 atomes de carbone, où ce reste peut être substitué une à quatre fois, de manière identique ou différente, par un atome d'halogène et/ou un radical alcoyle ayant 1 à 4 atomes de carbone, ou
$R^2$ représente un radical hétérocyclyle saturé ou insaturé, facultativement condensé à un benzène, avec 3 à 7 membres dans l'hétérocycle, parmi lesquels chaque fois 1 à 3 sont des hétéroatomes identiques ou différents, comme oxygène, azote ou soufre, où le reste peut être substitué une à trois fois de manière identique ou différente par un atome d'halogène, un radical alcoyle ayant 1 à 4 atomes de carbone, alcoxy ayant 1 à 4 atomes de carbone et/ou halogénoalcoyle ayant 1 à 4 atomes de carbone et 1 à 5 atomes d'halogène identiques ou différents, ou
$R^2$ représente un radical aryle ayant 6 à 10 atomes de carbone, où chacun de ces restes peut être substitué une à cinq fois, de manière identique ou différente, par
un atome d'halogène, un radical cyano, nitro, amino, hydroxyle, formyle, carboxyle, carbamoyle, thiocarbamoyle ;
un radical alcoyle, alcoxy, alcoylthio, alcoylsulfinyle ou alcoylsulfonyle chaque fois linéaire ou ramifié ayant chaque fois 1 à 6 atomes de carbone ;
un radical alcényle ou alcényloxy chaque fois linéaire ou ramifié ayant chaque fois 2 à 6 atomes de carbone ;
un radical halogénoalcoyle, halogénoalcoxy, halogénoalcoylthio, halogénoalcoylsulfinyle ou halogénoalcoylsulfonyle chaque fois linéaire ou ramifié ayant chaque fois 1 à 6 atomes de carbone et 1 à 13 atomes d'halogène identiques ou différents ;
un radical halogénoalcényle ou halogénoalcényloxy chaque fois linéaire ou ramifié ayant chaque fois 2 à 6 atomes de carbone et 1 à 13 atomes d'halogène identiques ou différents ;
un radical alcoylamino, dialcoylamino, alcoylcarbonyle, alcoylcarbonyloxy, alcoxycarbonyle, alcoylsulfonyloxy, hydroximinoalcoyle ou alcoximinoalcoyle chaque fois linéaire ou ramifié ayant chaque fois 1 à 6 atomes de carbone dans les unités alcoyle individuelles ;
un radical phényle, phénoxy, phénylthio, benzyloxy, benzylthio ou phényléthyloxy,
ou par un radical alcoylène relié deux fois, ayant 3 ou 4 atomes de carbone ou par un radical dioxyalcoylène relié deux fois, ayant 1 ou 2 atomes de carbone, où les deux restes cités en dernier lieu peuvent être eux-mêmes substitués une ou plusieurs fois, de manière identique ou différente par un atome d'halogène, un radical alcoyle linéaire ou ramifié ayant 1 à 4 atomes de carbone et/ou un radical halogénoalcoyle linéaire ou ramifié ayant 1 à 4 atomes de carbone et 1 à 9 atomes d'halogène identiques ou différents, et
n représente le nombre 0, 1, 2 ou 3,

caractérisé en ce que l'on fait réagir une N-acylamine racémique de formule :

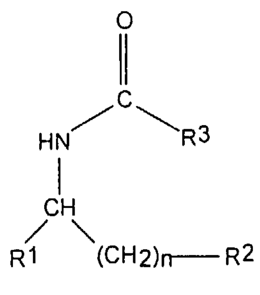

(II)

dans laquelle :

$R^1$, $R^2$ et n ont la signification donnée ci-dessus, et
$R^3$ représente un atome d'hydrogène, un radical amino, dialcoylamino ayant 1 à 6 atomes de carbone dans chaque unité alcoyle, alcoylthio ayant 1 à 6 atomes de carbone, alcoyle linéaire ou ramifié ayant 1 à 10 atomes de carbone ou alcoxy linéaire ou ramifié ayant 1 à 6 atomes de carbone, où les restes alcoyle ou les restes alcoxy peuvent être substitués une ou plusieurs fois, de manière identique ou différente par un atome d'halogène, un radical cyano, nitro, amino, hydroxyle, formyle, carboxyle, carbamoyle ;
un radical alcoxy, alcoylthio, alcoylsulfinyle ou alcoylsulfonyle chaque fois linéaire ou ramifié ayant chaque

fois 1 à 6 atomes de carbone ;
un radical alcényle ou alcényloxy chaque fois linéaire ou ramifié ayant chaque fois 2 à 6 atomes de carbone ;
un radical halogénoalcoxy, halogénoalcoylthio, halogénoalcoylsulfinyle ou halogénoalcoylsulfonyle chaque fois linéaire ou ramifié ayant chaque fois 1 à 6 atomes de carbone et 1 à 13 atomes d'halogène identiques ou différents ;
un radical alcoylamino, dialcoylamino, alcoylcarbonyle, alcoylcarbonyloxy, alcoxycarbonyle, alcoylsulfonyloxy, hydroximinoalcoyle ou alcoximinoalcoyle chaque fois linéaire ou ramifié ayant chaque fois 1 à 6 atomes de carbone dans les unités alcoyle individuelles ;
ou par un radical alcoylène relié deux fois, ayant 1 à 6 atomes de carbone ou par un radical dioxyalcoylène relié deux fois, ayant 1 à 4 atomes de carbone, où les deux restes cités en dernier lieu peuvent être eux-mêmes substitués une ou plusieurs fois, de manière identique ou différente par un atome d'halogène, un radical alcoyle linéaire ou ramifié ayant 1 à 4 atomes de carbone et/ou un radical halogénoalcoyle linéaire ou ramifié ayant 1 à 4 atomes de carbone et 1 à 9 atomes d'halogène identiques ou différents, ou
par un radical phényle, ou
$R^3$ représente un radical cycloalcoyle ayant_3 à 7 atomes de carbone, où ce reste peut être substitué une à quatre fois, de manière identique ou différente, par un atome d'halogène et/ou par un radical alcoyle ayant 1 à 4 atomes de carbone, ou
$R^3$ représente un radical cycloalcényle ayant 3 à 7 atomes de carbone, où ce reste peut être substitué une à quatre fois, de manière identique ou différente, par un atome d'halogène et/ou par un radical alcoyle ayant 1 à 4 atomes de carbone, ou
$R^3$ représente un radical hétérocyclyle saturé ou insaturé, avec 3 à 7 membres dans le cycle, parmi lesquels chaque fois 1 à 3 sont des hétéroatomes identiques ou différents, comme oxygène, azote ou soufre, où le reste peut être substitué une à trois fois de manière identique ou différente par un atome d'halogène, un radical alcoyle ayant 1 à 4 atomes de carbone, alcoxy ayant 1 à 4 atomes de carbone et/ou halogénoalcoyle ayant 1 à 4 atomes de carbone et 1 à 5 atomes d'halogène identiques ou différents, ou
$R^3$ représente un radical aryle ayant 6 à 10 atomes de carbone, où chacun de ces restes peut être substitué une à cinq fois, de manière identique ou différente, par un atome d'halogène, un radical cyano, alcoyle linéaire ou ramifié ayant 1 à 4 atomes de carbone,
un radical halogénoalcoyle linéaire ou ramifié ayant 1 à 4 atomes de carbone et 1 à 9 atomes d'halogène identiques ou différents,
un radical alcoxy ou alcoylthio linéaire ou ramifié ayant 1 à 4 atomes de carbone,
un radical halogénoalcoxy ou halogénoalcoylthio linéaire ou ramifié ayant 1 à 4 atomes de carbone et 1 à 9 atomes d'halogène identiques ou différents,
un radical phényle, phénoxy, phénylthio, benzyloxy, benzylthio ou phényléthyloxy,
ou par un radical alcoylène relié deux fois, ayant 3 ou 4 atomes de carbone ou par un radical dioxyalcoylène relié deux fois, ayant 1 ou 2 atomes de carbone, où les deux restes cités en dernier lieu peuvent être eux-mêmes substitués une ou plusieurs fois, de manière identique ou différente par un atome d'halogène, un radical alcoyle linéaire ou ramifié ayant 1 à 4 atomes de carbone et/ou un radical halogénoalcoyle linéaire ou ramifié ayant 1 à 4 atomes de carbone et 1 à 9 atomes d'halogène identiques ou différents,
avec une lipase de *Candida antartica*, une lipase de *Rhizopus niveus,* une lipase de *Pseudomonas* sp. ou une lipase de *Mucor javanicus,* en présence d'eau ainsi que facultativement en présence d'un agent de dilution organique à un pH situé dans l'intervalle allant de 3,0 à 10,0 et à une température située dans l'intervalle allant de 0°C à 80°C.

**2.** Procédé suivant la revendication 1, caractérisé en ce que l'on met en oeuvre comme N-acylamine racémique, le composé de formule :

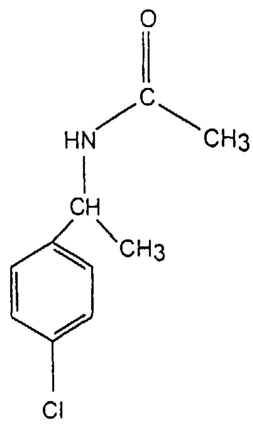

**3.** Procédé suivant la revendication 1, caractérisé en ce que l'on travaille à un pH situé dans l'intervalle allant de 4,0 à 9,0.

**4.** Procédé suivant la revendication 1, caractérisé en ce que l'on travaille à une température située dans l'intervalle allant de 20°C à 70°C.